(19) Europäisches Patentamt
European Patent Office
Office européen des brevets

(11) EP 4 481 379 A1

(12) EUROPEAN PATENT APPLICATION
published in accordance with Art. 153(4) EPC

(43) Date of publication:
25.12.2024 Bulletin 2024/52

(51) International Patent Classification (IPC):
G01N 33/20 (2019.01)   G01N 1/28 (2006.01)

(21) Application number: 23811451.6

(52) Cooperative Patent Classification (CPC):
G01N 1/28; G01N 21/17; G01N 33/20; G06T 7/00

(22) Date of filing: 30.03.2023

(86) International application number:
PCT/JP2023/013410

(87) International publication number:
WO 2023/228572 (30.11.2023 Gazette 2023/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 23.05.2022 JP 2022084131

(71) Applicant: JFE Steel Corporation
Tokyo 100-0011 (JP)

(72) Inventors:
• KIYOKANE Naoya
  Tokyo 100-0011 (JP)
• ISHIKAWA Shin
  Tokyo 100-0011 (JP)

(74) Representative: Hoffmann Eitle
Patent- und Rechtsanwälte PartmbB
Arabellastraße 30
81925 München (DE)

(54) **STRUCTURE PHOTOGRAPH EVALUATION METHOD, STRUCTURE PHOTOGRAPH EVALUATION DEVICE, IMAGING DEVICE, AND PROGRAM**

(57) A micrograph evaluating method, a micrograph evaluating device, an imaging device, and a program that can appropriately and efficiently evaluate the number of fields of view of micrographs of a metal material are provided. The micrograph evaluating method includes an input process (S11) of acquiring a plurality of micrographs taken at a defined magnification for two or more fields of view of a metal material; a phase classification process (S12) of classifying phases in the plurality of micrographs; a quantitative value calculation process (S13) of calculating quantitative values of the phases classified; a deviation calculation process (S 14) of calculating deviation of the quantitative values; a number of fields of view pass/fail determination process (S15) of determining a pass/fail result for a number of fields of view of the plurality of micrographs, based on the deviation; and an output process (S17) of outputting the pass/fail result.

*FIG. 2*

EP 4 481 379 A1

**Description**

TECHNICAL FIELD

The present disclosure relates to a micrograph evaluating method, a micrograph evaluating device, an imaging device, and a program. The present disclosure relates, in particular, to a micrograph evaluating method, a micrograph evaluating device, an imaging device, and a program, for micrographs of a metal material.

BACKGROUND

Typically, even when metal materials including steel have the same composition, properties are strongly dependent on metallic structure at the scale of an optical microscope or an electron microscope (for example, mm to μm scale).

For example, in the development of high strength steel sheets, methods to change the composition are used, such as solid solution strengthening by adding a solid-solution-strengthening element and strengthening by precipitation by adding a strengthening-by-precipitation element. Further, aside from such methods, other methods may be used to change the final metallic structure by changing heat treatment conditions with the same composition.

Thus, in the development of high strength steel sheets and the like, controlling not only the composition but also the metallic structure is important. Therefore, quantitatively evaluating metallic structure of a metal material by observation with an optical microscope or an electron microscope is important. For example, Patent Literature (PTL) 1 describes a method for preparing observation samples for observation with an electron microscope.

After carrying out sample preparation such as known polishing and etching methods on metal material such as a steel sheet, metallic structure can be observed using known imaging equipment such as an electron microscope, which typically allows classification of each phase because contrast is different for each phase. For example, regarding a steel material, when a representative steel sheet consisting of ferrite phase and pearlite phase is prepared as a sample by a known method and imaged by an optical microscope, ferrite phase is observed as having gray contrast and pearlite phase as having black contrast. Accordingly, ferrite phase and pearlite phase can be classified. Development is carried out by conducting observations of metallic structure using such methods and feeding back the results of the observations.

CITATION LIST

Patent Literature

PTL 1: JP 2009-287941 A

SUMMARY

(Technical Problem)

When a metal material is newly developed, appropriate evaluation of metallic structure is required. However, an appropriate number of fields of view for observing metallic structure is often unclear. In such cases, conventionally, the number of fields of view has been determined subjectively by an observer. Therefore, the metallic structure may be evaluated using micrographs with an insufficient number of fields of view, metallic structure may not be appropriately evaluated, and correlation between metallic structure and a material property may be misinterpreted. When a production process is determined based on a misinterpreted correlation, there is a risk of being unable to produce a product having a desired property. Further, in an attempt to avoid misinterpretation, an observer may take more micrographs with more fields of view than necessary, reducing work efficiency.

In view of such circumstances, it would be helpful to provide a micrograph evaluating method, a micrograph evaluating device, an imaging device, and a program that can appropriately and efficiently evaluate the number of fields of view of micrographs of a metal material.

(Solution to Problem)

(1) A micrograph evaluating method according to an embodiment of the present disclosure comprises:

an input process of acquiring a plurality of micrographs taken at a defined magnification for two or more fields of view of a metal material;
a phase classification process of classifying phases in the plurality of micrographs;
a quantitative value calculation process of calculating quantitative values of the phases classified;
a deviation calculation process of calculating deviation of the quantitative values;
a number of fields of view pass/fail determination process of determining a pass/fail result for a number of fields of view of the plurality of micrographs, based on the deviation; and
an output process of outputting the pass/fail result.

(2) A micrograph evaluating method according to an embodiment of the present disclosure comprises:

an input process of acquiring a plurality of micrographs taken at a defined magnification for two or more fields of view of a metal material;
a phase classification process of classifying phases in the plurality of micrographs;
a quantitative value calculation process of calculating quantitative values of the phases classified;
a deviation calculation process of calculating deviation of the quantitative values;
a number of fields of view pass/fail determination process of determining a pass/fail result for a number of fields of view of the plurality of micrographs, based on the deviation; and
a statistical value calculation process of calculating a statistical value based on the quantitative values of the phases; and
an output process of outputting the statistical value.

(3) A micrograph evaluating method according to an embodiment of the present disclosure comprises:

an input process of acquiring a plurality of micrographs taken at two or more magnifications for two or more fields of view of a metal material;
a phase classification process of classifying phases in the plurality of micrographs;
a quantitative value calculation process of calculating quantitative values of the phases classified;
a magnification determination process of determining a representative magnification based on the quantitative values; and
a deviation calculation process of calculating deviation of the quantitative values for a set of the plurality of micrographs corresponding to the representative magnification;
a number of fields of view pass/fail determination process of determining a pass/fail result for a number of fields of view of the set of the plurality of micrographs, based on the deviation; and
a statistical value calculation process of calculating a statistical value based on the quantitative values of the phases; and
an output process of outputting the statistical value.

(4) The micrograph evaluating method according to (2) or (3), as an embodiment of the present disclosure, wherein the output process outputs the pass/fail result along with the statistical value.
(5) The micrograph evaluating method according to any one of (1) to (3), as an embodiment of the present disclosure, wherein
the quantitative values are at least one of area fraction, ellipsoid size, Feret diameter, average diameter, circularity, or number density.
(6) A micrograph evaluating device according to an embodiment of the present disclosure comprises:

an input interface configured to acquire a plurality of micrographs taken at a defined magnification for two or more fields of view of a metal material;
a phase classification unit configured to classify phases in the plurality of micrographs;
a quantitative value calculation unit configured to calculate quantitative values of the phases classified;
a deviation calculation unit configured to calculate deviation of the quantitative values;
a number of fields of view pass/fail determination unit configured to determine a pass/fail result for a number of fields of view of the plurality of micrographs, based on the deviation; and
an output interface configured to output the pass/fail result.

(7) A micrograph evaluating device according to an embodiment of the present disclosure comprises:

an input interface configured to acquire a plurality of micrographs taken at a defined magnification for two or more fields of view of a metal material;

EP 4 481 379 A1

a phase classification unit configured to classify phases in the plurality of micrographs;
a quantitative value calculation unit configured to calculate quantitative values of the phases classified;
a deviation calculation unit configured to calculate deviation of the quantitative values;
a number of fields of view pass/fail determination unit configured to determine a pass/fail result for a number of fields of view of the plurality of micrographs, based on the deviation; and
a statistical value calculation unit configured to calculate a statistical value based on the quantitative values of the phases; and
an output interface configured to output the statistical value.

(8) A micrograph evaluating device according to an embodiment of the present disclosure comprises:

an input interface configured to acquire a plurality of micrographs taken at two or more magnifications for two or more fields of view of a metal material;
a phase classification unit configured to classify phases in the plurality of micrographs;
a quantitative value calculation unit configured to calculate quantitative values of the phases classified;
a magnification determination unit configured to determine a representative magnification based on the quantitative values; and
a deviation calculation unit configured to calculate deviation of the quantitative values for a set of the plurality of micrographs corresponding to the representative magnification;
a number of fields of view pass/fail determination unit configured to determine a pass/fail result for a number of fields of view of the set of the plurality of micrographs, based on the deviation; and
a statistical value calculation unit configured to calculate a statistical value based on the quantitative values of the phases; and
an output interface configured to output the statistical value.

(9) An imaging device according to an embodiment of the present disclosure, configured to
take the plurality of micrographs acquired by the micrograph evaluating device according to any one of (6) to (8).
(10) The imaging device according to (9), as an embodiment of the present disclosure, wherein
the imaging device is an optical microscope or a scanning electron microscope.
(11) A program according to an embodiment of the present disclosure is configured to cause a computer to function as

an input interface configured to acquire a plurality of micrographs taken at a defined magnification for two or more fields of view of a metal material;
a phase classification unit configured to classify phases in the plurality of micrographs;
a quantitative value calculation unit configured to calculate quantitative values of the phases classified;
a deviation calculation unit configured to calculate deviation of the quantitative values;
a number of fields of view pass/fail determination unit configured to determine a pass/fail result for a number of fields of view of the plurality of micrographs, based on the deviation; and
an output interface configured to output the pass/fail result.

(12) A program according to an embodiment of the present disclosure is configured to cause a computer to function as

an input interface configured to acquire a plurality of micrographs taken at a defined magnification for two or more fields of view of a metal material;
a phase classification unit configured to classify phases in the plurality of micrographs;
a quantitative value calculation unit configured to calculate quantitative values of the phases classified;
a deviation calculation unit configured to calculate deviation of the quantitative values;
a number of fields of view pass/fail determination unit configured to determine a pass/fail result for a number of fields of view of the plurality of micrographs, based on the deviation; and
a statistical value calculation unit configured to calculate a statistical value based on the quantitative values of the phases; and
an output interface configured to output the statistical value.

(13) A program according to an embodiment of the present disclosure is configured to cause a computer to function as

an input interface configured to acquire a plurality of micrographs taken at two or more magnifications for two or more fields of view of a metal material;
a phase classification unit configured to classify phases in the plurality of micrographs;

4

a quantitative value calculation unit configured to calculate quantitative values of the phases classified;

a magnification determination unit configured to determine a representative magnification based on the quantitative values; and

a deviation calculation unit configured to calculate deviation of the quantitative values for a set of the plurality of micrographs corresponding to the representative magnification;

a number of fields of view pass/fail determination unit configured to determine a pass/fail result for a number of fields of view of the set of the plurality of micrographs, based on the deviation; and

a statistical value calculation unit configured to calculate a statistical value based on the quantitative values of the phases; and

an output interface configured to output the statistical value.

(Advantageous Effect)

According to the present disclosure, it is possible to provide a micrograph evaluating method, a micrograph evaluating device, an imaging device, and a program that can appropriately and efficiently evaluate the number of fields of view of micrographs of a metal material.

BRIEF DESCRIPTION OF THE DRAWINGS

In the accompanying drawings:

FIG. 1 is a schematic diagram illustrating an example configuration of a micrograph evaluating system including a micrograph evaluating device according to an embodiment of the present disclosure;

FIG. 2 is a flowchart illustrating processing of a micrograph evaluating method according to an embodiment of the present disclosure;

FIG. 3 is a schematic diagram illustrating an example configuration of a micrograph evaluating system including a micrograph evaluating device according to an embodiment of the present disclosure;

FIG. 4 is a flowchart illustrating processing of a micrograph evaluating method according to an embodiment of the present disclosure;

FIG. 5 is a schematic diagram illustrating an example configuration of a micrograph evaluating system including a micrograph evaluating device according to an embodiment of the present disclosure;

FIG. 6 is a flowchart illustrating processing of a micrograph evaluating method according to an embodiment of the present disclosure;

FIG. 7 illustrates micrographs used in an Example; and

FIG. 8 illustrates the micrographs of FIG. 7 after a phase classification process.

DETAILED DESCRIPTION

Hereinafter, with reference to the drawings, a micrograph evaluating method, a micrograph evaluating device, an imaging device, and a program are described according to embodiments of the present disclosure. In each drawing, identical or equivalent parts are marked with the same reference sign. In description of the following embodiments, description of identical or equivalent parts is omitted or simplified as appropriate.

[First embodiment]

(Micrograph evaluating system)

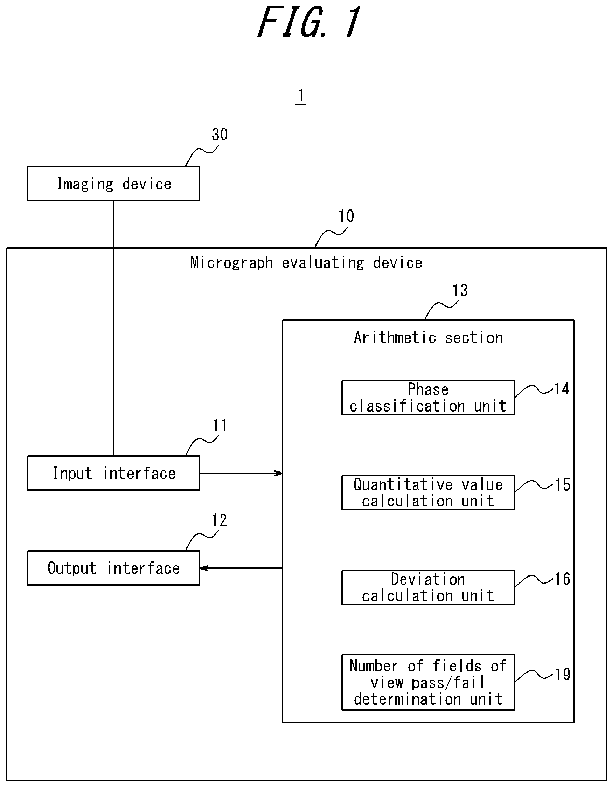

FIG. 1 is a block diagram of the micrograph evaluating system 1 including the micrograph evaluating device 10 according to a first embodiment. The micrograph evaluating system 1 includes the micrograph evaluating device 10 and an imaging device 30. The micrograph evaluating device 10 includes an input interface 11, an output interface 12, and an arithmetic section 13. The arithmetic section 13 includes a phase classification unit 14, a quantitative value calculation unit 15, a deviation calculation unit 16, and a number of fields of view pass/fail determination unit 19.

(Imaging device)

The imaging device 30 takes a plurality of micrographs of a metal material to be acquired by the micrograph evaluating device 10. The imaging device 30 may be, for example, an optical microscope or a scanning electron microscope, but is not limited to these examples and may be any device that has a function of imaging microstructure of a metal material.

(Micrograph evaluating device)

The micrograph evaluating device 10 executes processing for evaluating a plurality of micrographs of a metal material taken by the imaging device 30. Here, the metal material is a steel material or a metal material having a plurality of metallic phases. Evaluation executed by the micrograph evaluating device 10 includes an evaluation of the number of fields of view of a plurality of micrographs. The evaluation of the number of fields of view of a plurality of micrographs is an evaluation of whether the number of fields of view is sufficient to quantitatively evaluate the metallic structure. The micrograph evaluating device 10 may be configured to directly execute microstructure evaluation and may be configured to determine a property of micrographs. The property of micrographs to be determined may be, for example, a pass/fail result regarding the number of fields of view. According to the present embodiment, the micrograph evaluating device 10 determines a pass/fail result of the number of fields of view of micrographs. Here, the number of fields of view refers to the number of micrographs of different fields of view. The number of fields of view and the number of micrographs may differ, but according to the embodiments of the present disclosure, each micrograph has a different field of view, and the number of fields of view is described as equal to the number of micrographs of a metal material imaged.

(Input interface)

The input interface 11 is an input interface of the micrograph evaluating device 10 that acquires data required for processing evaluating a plurality of micrographs. According to the present embodiment, the input interface 11 acquires a plurality of micrographs taken at a defined magnification for two or more fields of view of a metal material. The data format for the plurality of micrographs may be a commonly used image data format such as TIFF, BMP, or the like.

(Output interface)

The output interface 12 is an output interface of the micrograph evaluating device 10 that outputs a micrograph property (for example, a pass/fail result determined and statistical value described later). The output interface 12 may transmit information such as a micrograph property to another device or the like. Further, the output interface 12 may display a micrograph property or the like on a display device such as any of various displays or the like. According to the present embodiment, the output interface 12 outputs a pass/fail result for the number of fields of view for a plurality of micrographs.

(Arithmetic section)

The arithmetic section 13 executes calculations to evaluate a plurality of micrographs. Further, the arithmetic section 13 may function as a controller that controls the micrograph evaluating device 10 as a whole. The arithmetic section 13 may be one or more processors. Each processor may be, but is not limited to, a general-purpose processor or a dedicated processor specialized for particular processing, and may be any processor.

As described above, according to the present embodiment, the arithmetic section 13 includes the phase classification unit 14, the quantitative value calculation unit 15, the deviation calculation unit 16, and the number of fields of view pass/fail determination unit 19. The functions of the phase classification unit 14, the quantitative value calculation unit 15, the deviation calculation unit 16, and the number of fields of view pass/fail determination unit 19 may be realized by software. For example, one or more programs may be stored in a storage device accessible by the arithmetic section 13. A program stored in the storage device, when read by the arithmetic section 13 that is a processor, may cause the arithmetic section 13 to function as the phase classification unit 14, the quantitative value calculation unit 15, the deviation calculation unit 16, and the number of fields of view pass/fail determination unit 19.

(Phase classification unit)

The phase classification unit 14 classifies phases in a plurality of micrographs. The details of processing executed by the phase classification unit 14 are described later.

(Quantitative value calculation unit)

The quantitative value calculation unit 15 calculates quantitative values for the phases classified by the phase classification unit 14. The details of processing executed by the quantitative value calculation unit 15 are described later.

(Deviation calculation unit)

The deviation calculation unit 16 calculates deviation of the quantitative values calculated by the quantitative value calculation unit 15. The details of processing executed by the deviation calculation unit 16 are described later.

(Number of fields of view pass/fail determination unit)

The number of fields of view pass/fail determination unit 19 makes a pass/fail determination on the number of fields of view of a plurality of micrographs based on the deviation calculated by the deviation calculation unit 16. The details of processing executed by the number of fields of view pass/fail determination unit 19 are described later.

Here, the micrograph evaluating device 10 is not limited to a specific device, but may be realized by a computer as an example. The computer may be a commercially available, general-purpose computer, for example. The computer may, for example, include a storage device, such as memory and a hard disk drive, a CPU, and input/output devices. The arithmetic section 13 may be realized by a CPU. The program to be read by the arithmetic section 13 may be stored in a storage device. The input interface 11 and the output interface 12 may be realized by input/output devices.

(Micrograph evaluating method)

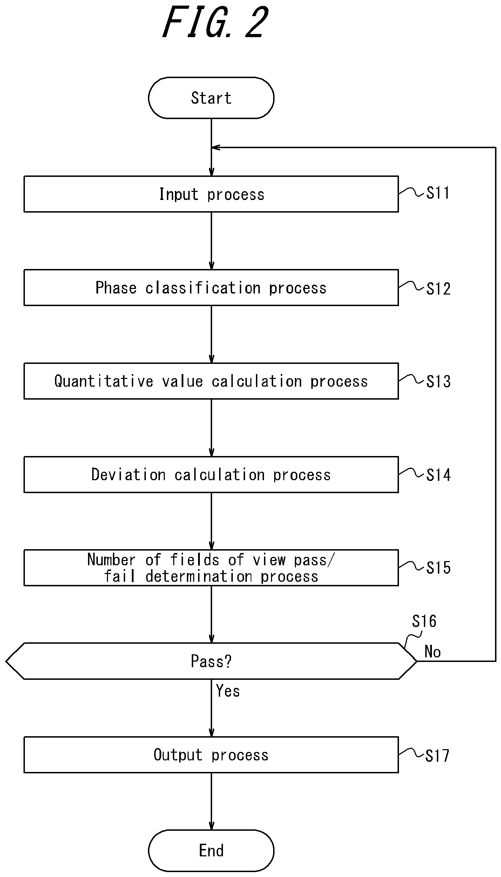

FIG. 2 is a flowchart illustrating processing of the micrograph evaluating method executed by the micrograph evaluating device 10 according to the present embodiment. In summary, the micrograph evaluating method includes, in this order: an input process of acquiring a plurality of micrographs taken at a defined magnification for two or more fields of view of a metal material; a phase classification process of classifying phases in the plurality of micrographs; a quantitative value calculation process of calculating quantitative values of the phases classified; a deviation calculation process of calculating deviation of the quantitative values; a number of fields of view pass/fail determination process of determining a pass/fail result for a number of fields of view of the plurality of micrographs, based on the deviation; and an output process of outputting the pass/fail result. Here, when the micrograph evaluating method, in the number of fields of view pass/fail determination, does not result in a pass (that is, results in a fail), processing is executed again with the addition of a micrograph acquired in the input process.

(Input process)

The input process is a process in which the input interface 11 acquires a plurality of micrographs taken at a defined magnification for two or more fields of view of a metal material (step S11).

After sample preparation of a surface of a metal material (sample) by a known method, such as polishing, a micrograph is preferably taken by a known imaging device such as an optical microscope or a scanning electron microscope. Sample preparation is typically carried out by rough polishing followed by finish polishing. Rough polishing and finish polishing may be carried out by known methods. Rough polishing by a known method may, for example, be polishing to remove scratches visible at the naked eye level, using a commercially available paper file coated with abrasive grains on paper (such as emery paper). Further, finish polishing by a known method is polishing carried out with a 0.05 $\mu$m to 2 $\mu$m abrasive until the sample has a mirror-like finish. As an abrasive, a known abrasive such as diamond, silica, or the like may be used. Mirror polishing is carried out until polishing scratches are no longer noticeable when observed under an optical microscope at 10× to 500× magnification. When many polishing scratches remain, they may cause errors in phase classification during the phase classification process, and therefore carrying out polishing with as few scratches as possible is desirable. After mirror polishing is carried out, etching with nital or the like may be carried out to clarify phase contrast. Etching may be omitted, such as for a sample that can be phase classified without etching.

The number of fields of view for the micrographs is two or more. The greater the number of fields of view, the less the variation in quantitative value of phases at each magnification. The fields of view are preferably determined randomly. Further, two or more fields of view may be acquired by taking a series of images while changing the field of view slightly. Further, when the imaging device 30 includes a function that automatically determines the field of view, such as by using random numbers via a program, such a function may be used.

The magnification of micrographs is not limited to a specific magnification, and may be any magnification at which a target microstructure can be observed. For example, when observing dual phase steel (DP steel) consisting of ferrite phase and martensite phase, magnification is preferably selected in a range from 500× to 3000×. For example, when observing a coated or plated layer of a coated or plated steel sheet, magnification is preferably selected in a range from 1000× to 5000×.

(Phase classification process)

The phase classification process is a process in which the phase classification unit 14 classifies phases in a plurality of micrographs (step S12).

The contrast of phases generally differ from each other, and therefore micrographs can be classified into phases. For example, phases are identified in a micrograph, and each phase is labeled with a different color or the like for classification. Phase classification may be performed by identifying phases with the naked eye and classifying by hand painting, by using binarization of image luminance values, or by advanced image interpretation methods. Application of a classification method that is as precise as possible is preferred.

However, phase classification by hand painting lacks objectivity. Therefore, binarization or advanced image interpretation methods are preferably used. As exemplified below, advanced image interpretation methods are more preferably used.

In an advanced image interpretation method, a plurality of regions of each phase are specified from a captured micrograph, feature values are calculated from image luminance values in the specified regions, and classification is performed using a method such as a random forest model, a neural network model, or a support vector machine that repeatedly converts calculated feature values into N values. One or more of the following eight specific examples (C1 to C8) is preferably used for feature values.

(C1: constant feature value)

A constant feature value is a feature value that indicates the luminance value of a micrograph itself.

(C2: mean feature value)

A mean feature value is a feature value that indicates the mean value of luminance values in a defined range of a micrograph. That is, a mean feature value is the result of taking a defined range "(number of pixels x) × (number of pixels y)" from each phase of a micrograph and averaging luminance values within the range. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

Here, "noise in a micrograph" indicates, for example, a portion of a micrograph in which the luminance value suddenly increases. Further, the number of pixels x and y being greater than noise indicates making the numbers greater than noise width.

(C3: Gaussian feature value)

A Gaussian feature value is a feature value that indicates an average of luminance values in a defined range of a micrograph, with greater weight the closer to the center. That is, a Gaussian feature value is the result of taking a defined range "(number of pixels x) × (number of pixels y)" from each phase of a micrograph and taking averaged luminance values within the range while weighting central pixels more heavily. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C4: median feature value)

A median feature value is a feature value that indicates the median value of luminance values in a defined range of a micrograph. That is, a median feature value is the result of taking a defined range "(number of pixels x) × (number of pixels y)" from each phase of a micrograph and taking a median luminance value within the range. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C5: max feature value)

A max feature value is a feature value that indicates the maximum value of luminance values in a defined range of a micrograph. That is, a max feature value is the result of taking a defined range "(number of pixels x) × (number of pixels y)" from each phase of a micrograph and taking the maximum luminance value within the range. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C6: min feature value)

A min feature value is a feature value that indicates the minimum value of luminance values in a defined range of a micrograph. That is, a min feature value is the result of taking a defined range "(number of pixels x) × (number of pixels y)" from each phase of a micrograph and taking the minimum luminance value within the range. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C7: derivative feature value)

A derivative feature value is the result of taking a defined range "(number of pixels x) × (number of pixels y)" from each phase of a micrograph and calculating differential values in the x direction and the y direction relative to end pixels. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

(C8: derivative sum feature value)

A derivative sum feature value is the convolution of a derivative feature value by calculating the mean feature value, the Gaussian feature value, the median feature value, the max feature value, and the min feature value for the derivative feature value described above. Here, "number of pixels x" and "number of pixels y" may be the same size or different sizes. The area of "(number of pixels x) × (number of pixels y)" need not be rectangular, but is preferably a circular area when a micrograph is circular in shape, and may be calculated for a plurality of numbers of pixels x and y. Lower limits of "number of pixels x" and "number of pixels y" are preferably, for example, in a range that includes a size greater than noise in a micrograph and includes a size less than 1/2 the smaller crystal grain size of the plurality of phases of the metallic structure. When upper limits are too large, the mean feature value may be affected by grain boundaries or other adjacent phases, and therefore are preferably in a range that includes a size less than 1/2 the crystal grain size of the larger crystal grain size.

Here, when an image is analyzed using an advanced image interpretation method, a boundary (outer circumferential portion) may be truncated for accurate analysis.

(Quantitative value calculation process)

The quantitative value calculation process is a process in which the quantitative value calculation unit 15 calculates quantitative values for the phases classified by the phase classification unit 14. For example, at least one of the following (1) to (6) is calculated as quantitative values.

In the specific examples described below, quantitative values (1) through (5) are calculated for each crystal grain of a specified phase after classification, and therefore multiple values are acquired even for a single micrograph. Further, aside from the specific examples described below, when another parameter can be used to quantify a micrograph, such a parameter may be used.

(1) Area fraction

Area fraction is calculated as the ratio of the area of each crystal grain of a specified phase to the area of the entire micrograph in each micrograph. Area fraction for each crystal grain is calculated according to the following expression.

[Math. 1]

$$f = \frac{A_i}{A_{sum}}$$

Here, f is area fraction for each crystal grain. $A_i$ is area for each crystal grain. $A_{sum}$ is area of the entire micrograph. Further, the area fraction for the specified phase per micrograph is calculated by calculating the sum of the area fraction of each crystal grain for the specified phase.

(2) Ellipsoid size

Ellipsoid size is calculated by approximating an ellipsoid to the shape of each crystal grain of the specified phase in each micrograph. The ellipsoid size is at least one of the major axis length, minor axis length, or aspect ratio of the approximated ellipsoid. Further, the average ellipsoid size for the specified phase per micrograph is calculated by calculating the average ellipsoid size of each crystal grain for the specified phase.

(3) Feret diameter

In each micrograph, after drawing a straight line from a boundary of each crystal grain of the specified phase, the Feret diameter with the greatest linear distance is calculated. Further, the average Feret diameter for the specified phase per micrograph is calculated by calculating the average Feret diameter calculated for each crystal grain.

(4) Average diameter

In each micrograph, the area of each crystal grain of the specified phase is determined and crystal grain average diameter is calculated by taking the square root of the area. Further, the average diameter for the specified phase per micrograph is calculated by calculating the average value from the average diameter calculated for each crystal grain for the specified phase.

(5) Circularity

In each micrograph, circularity of each crystal grain is calculated according to the following expression, by determining the area and circumference of each crystal grain of the specified phase.

[Math. 2]

$$C = 4\pi \times \frac{S}{p^2}$$

Here, C is circularity. S is area. p is circumference. When a crystal grain is a true circle, the circularity is 1.0. Conversely, the further the crystal grain shape is from a circle, the smaller the circularity becomes, below 1.0. Further, the average circularity for the specified phase per micrograph is calculated by calculating the average value from the circularity calculated for each crystal grain for the specified phase.

(6) Number density

In each micrograph, the number density per micrograph is calculated by counting the number of crystal grains of the specified phase and dividing the number of crystal grains by the area of the entire micrograph.

Preferably one or more of (1) to (6) are used as the quantitative values, and more preferably two or more are used. In particular, when determining a pass/fail result for a number of fields of view, the quantitative values preferably include the area fraction of (1).

(Deviation calculation process)

In the deviation calculation process (step S14), deviation is calculated for each field of view. The deviation calculation unit 16 calculates an average value $N_i$ of the quantitative values i of micrographs for all fields of view for the specified phase. Further, the deviation calculation unit 16 calculates an average value $\mu_{ij}$ of the quantitative values i of a micrograph for field of view j for the specified phase. The deviation calculation unit 16 calculates a normalized quantitative value i ($\mu_{ij}/N_i$) for field of view j by dividing the average value $\mu_{ij}$ of the quantitative values i of the micrograph for field of view j by the average value $N_i$ of the quantitative values i of the micrographs for all fields of view. Further, the deviation calculation unit 16 calculates an average value of deviation of the quantitative value i. The average value of deviation is calculated by dividing the sum of "the absolute value of each normalized quantitative value i minus 1" by the number of fields of view (number of micrographs) and is represented by the following expression.

[Math. 3]

$$S_i = \frac{\sum_j^n \left| \frac{\mu_{ji}}{N_i} - 1 \right|}{n}$$

Here, $S_i$ is the average value of deviations of the quantitative values i. n is the number of fields of view (number of micrographs).

Further, normalized quantitative value i is denoted by the prefix "normalized". For example, "normalized area fraction" means area fraction normalized by the deviation calculation process described above. Similarly, "normalized Feret diameter" and "normalized circularity" mean Feret diameter when normalized and circularity when normalized, respectively.

(Number of fields of view pass/fail determination process)

The number of fields of view pass/fail determination process (step S 15) is a process in which the number of fields of view pass/fail determination unit 19 determines pass or fail for the number of fields of view of micrographs, based on the deviations of the quantitative values i. The number of fields of view pass/fail determination unit 19 first determines whether the number of fields of view (number of micrographs) allows appropriate evaluation of microstructure (pass/fail) based on average values $S_i$ of the deviations calculated by the deviation calculation unit 16. For example, when the average values $S_i$ of the deviations of the quantitative values are all a threshold value or less, the number of fields of view pass/fail determination unit 19 determines that the number of fields of view is sufficient (Yes in step S16) and an additional micrograph need not be taken. The threshold value may be 0.1 as an example, but is not limited to a specific value and may be set according to the type of metal being targeted, for example. For example, when even one of the average values $S_i$ of the deviations of the quantitative values is larger than the threshold value, the deviation of the quantitative values is large and a relationship between the quantitative values and a material property cannot be accurately determined, and therefore the number of fields of view pass/fail determination unit 19 determines a fail result (No in step S16). When determining a fail result, the number of fields of view pass/fail determination unit 19 returns processing to the input process (step S11) after adding a micrograph acquired in the input process, and causes the series of processing to be executed again.

(Output process)

When the number of fields of view pass/fail determination unit 19 determines a pass result (Yes in step S16), the output process is executed (step S17). The output process is a process in which the output unit 12 outputs the pass/fail result of the number of fields of view. Here, in the flowchart example in FIG. 2, the output process is executed only in the case of a pass, but the output process may also be executed after a defined number of fails. For example, when the processing from step S16 back to step S11 is repeated a defined number of times (five times as an example), and the number of fields of view pass/fail determination unit 19 determines a fail result, the output process may be executed to output the fail result.

In this way, the micrograph evaluating method executed by the micrograph evaluating device 10 according to the present embodiment can appropriately and efficiently evaluate the number of fields of view of micrographs of a metal material. A result of evaluation is fed back to an observer to determine an appropriate number of fields of view for observing metallic structure, independently of subjectivity of the observer. Further, an observer does not have to take unnecessarily large numbers of micrographs while changing the field of view, and therefore work efficiency can be improved.

[Second embodiment]

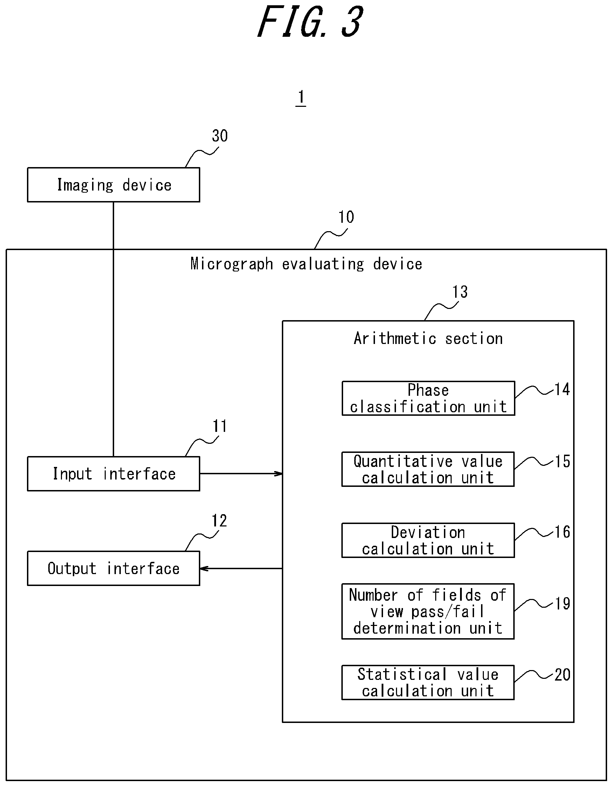

FIG. 3 is a block diagram of the micrograph evaluating system 1 including the micrograph evaluating device 10 according to a second embodiment. The micrograph evaluating device 10 according to the present embodiment outputs a statistical value based on the quantitative values of phases when executing micrograph evaluation. The output of such a statistical value outside of the micrograph evaluating device 10 allows for verification or secondary pass/fail judgment and the like, regarding the evaluation executed by the micrograph evaluating device 10. The micrograph evaluating device 10 according to the present embodiment includes the components of the micrograph evaluating device 10 according to the first embodiment, and further includes a statistical value calculation unit 20. To avoid duplicate description, only configurations that differ from those of the first embodiment are described below.

(Statistical value calculation unit)

The statistical value calculation unit 20 calculates a statistical value based on the quantitative values of the phases. According to the present embodiment, the statistical value calculation unit 20 calculates a statistical value based on the quantitative values of all of the plurality of micrographs entered into the input interface 11. A statistical value may include, but is not limited to, mean, median, standard deviation, and the like. According to the present embodiment, the statistical value calculation unit 20 calculates a statistical value when the number of fields of view pass/fail determination unit 19 determines that the number of fields of view has passed. However, this is not a limitation, and the statistical value calculation unit 20 may calculate a statistical value after a defined number of fail results. Further, the output interface 12 may output a pass/fail result along with a statistical value calculated by the statistical value calculation unit 20.

Further, according to the present embodiment, as per the first embodiment, the input interface 11 acquires a plurality of micrographs taken at a defined magnification for two or more fields of view of a metal material. A program stored in the storage device, when read by the arithmetic section 13 that is a processor, may cause the arithmetic section 13 to function as the phase classification unit 14, the quantitative value calculation unit 15, the deviation calculation unit 16, the number of fields of view pass/fail determination unit 19, and the statistical value calculation unit 20.

(Micrograph evaluating method)

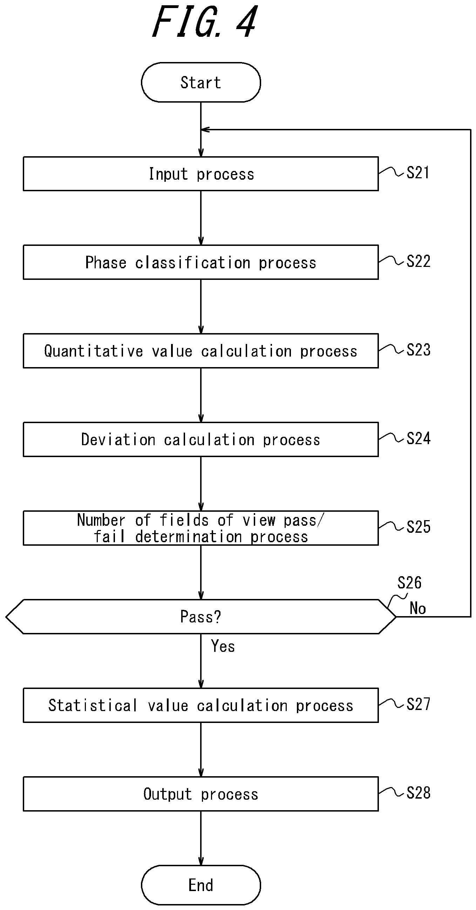

FIG. 4 is a flowchart illustrating processing of the micrograph evaluating method executed by the micrograph evaluating device 10 according to the present embodiment. In summary, the micrograph evaluating method includes, in this order: an input process of acquiring a plurality of micrographs taken at a defined magnification for two or more fields of view of a metal material; a phase classification process of classifying phases in the plurality of micrographs; a quantitative value calculation process of calculating quantitative values of the phases classified; a deviation calculation process of calculating deviation of the quantitative values; a number of fields of view pass/fail determination process of determining a pass/fail result for a number of fields of view of the plurality of micrographs, based on the deviation; a statistical value calculation process of calculating a statistical value based on the quantitative values of the phases; and an output process of outputting the statistical value. Here, when the micrograph evaluating method determines a fail result in the number of fields of view pass/fail determination process, these processes are executed again with the addition of a micrograph acquired in the input process.

The input process (step S21), the phase classification process (step S22), the quantitative value calculation process (step S23), the deviation calculation process (step S24), and the number of fields of view pass/fail determination process (step S25) are each the same as the process with the same name according to the first embodiment. As in the first embodiment, when the number of fields of view pass/fail determination process determines a fail result (No in step S26), processing returns to the input process (step S21) and the series of processing is executed again.

(Statistical value calculation process)

When the number of fields of view pass/fail determination process determines a pass result (Yes in step S26), the statistical value calculation process (step S27) is executed. In the statistical value calculation process according to the present embodiment, the statistical value calculation unit 20 calculates the average and standard deviation for all fields of view of each quantitative value calculated in the quantitative value calculation process.

After the statistical value calculation process is executed, the output process is executed (step S28). The output process is a process of outputting the statistical value calculated by the statistical value calculation unit 20. Here, as described above, the output process may output a pass/fail result along with the statistical value.

In this way, the micrograph evaluating method executed by the micrograph evaluating device 10 according to the present embodiment can appropriately and efficiently evaluate the number of fields of view of micrographs of a metal material. A result of evaluation is fed back to an observer to determine an appropriate number of fields of view for observing metallic structure, independently of subjectivity of the observer. Further, an observer does not have to take unnecessarily large numbers of micrographs while changing the field of view, and therefore work efficiency can be improved. Further, the output of a statistical value outside of the micrograph evaluating device 10 allows for verification or secondary pass/fail judgment and the like, regarding the evaluation executed by the micrograph evaluating device 10.

[Third embodiment]

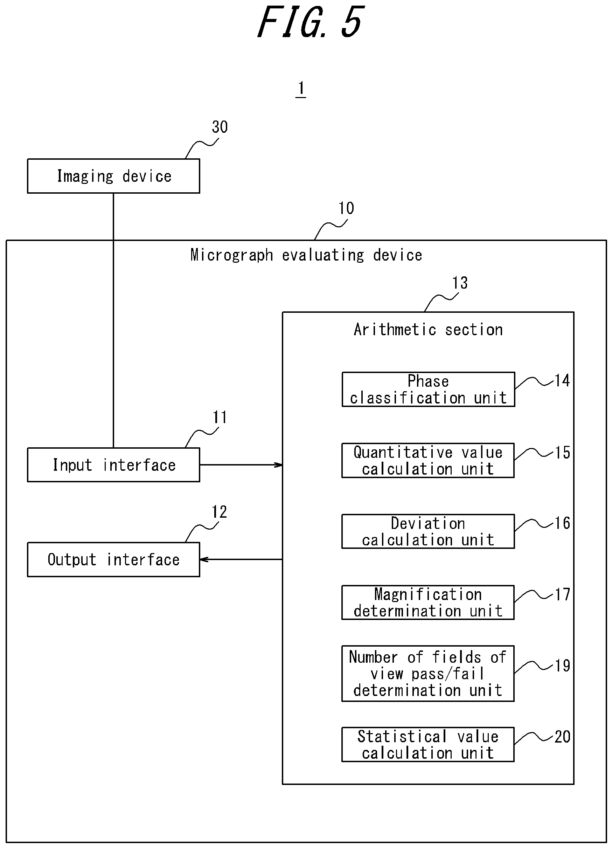

FIG. 5 is a block diagram of the micrograph evaluating system 1 including the micrograph evaluating device 10 according to a third embodiment. The micrograph evaluating device 10 according to the present embodiment determines a representative magnification before evaluating the number of fields of view. The micrograph evaluating device 10 according to the present embodiment includes the components of the micrograph evaluating device 10 according to the second embodiment, and further includes a magnification determination unit 17. To avoid duplicate description, only configurations that differ from those of the first embodiment and the second embodiment are described below.

According to the present embodiment, the input interface 11 acquires a plurality of micrographs taken at two or more magnifications for two or more fields of view of a metal material. That is, unlike the first embodiment and the second embodiment, the input interface 11 acquires a plurality of micrographs without the magnification being determined. Here, the greater the number of levels of magnification, the more precise the relationship between acquired magnification and quantitative values of the phases for each magnification, and the easier it becomes to determine an appropriate magnification. The number of levels of magnification of micrographs is preferably 3 or more. The number of levels of magnification of micrographs is more preferably 5 or more. Magnification is preferably selected randomly.

(Magnification determination unit)

The magnification determination unit 17 determines a representative magnification based on quantitative values calculated by the quantitative value calculation unit 15. The details of processing executed by the magnification determination unit 17 are described later.

Here, a program stored in the storage device, when read by the arithmetic section 13 that is a processor, may cause the arithmetic section 13 to function as the phase classification unit 14, the quantitative value calculation unit 15, the deviation calculation unit 16, the magnification determination unit 17, the number of fields of view pass/fail determination unit 19, and the statistical value calculation unit 20.

(Micrograph evaluating method)

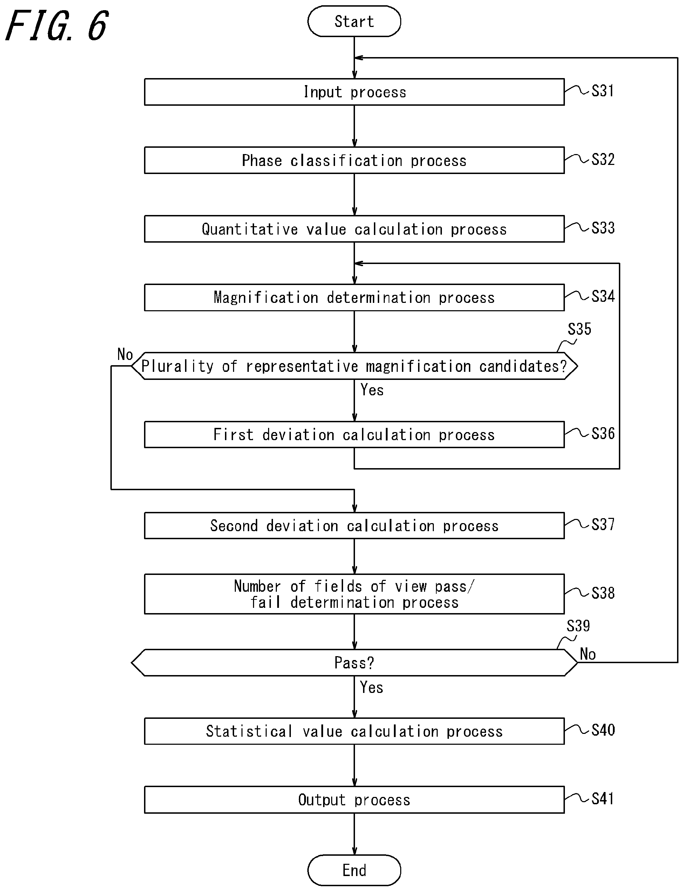

FIG. 6 is a flowchart illustrating processing of the micrograph evaluating method executed by the micrograph evaluating device 10 according to the present embodiment. In summary, the micrograph evaluating method includes: an input process of acquiring a plurality of micrographs taken at two of more magnifications for two or more fields of view of a metal material; a phase classification process of classifying phases in the plurality of micrographs; a quantitative value calculation process of calculating quantitative values of the phases classified; a magnification determination process of determining a representative magnification based on the quantitative values; a deviation calculation process (second deviation calculation process) of calculating deviation of the quantitative values, for a set of the plurality of micrographs corresponding to the representative magnification; a number of fields of view pass/fail determination process of determining a pass/fail result for a number of fields of view of the set of the plurality of micrographs, based on the deviation; a statistical value calculation process of calculating a statistical value based on the quantitative values of the phases; and an output process of outputting the statistical value. Here, when the micrograph evaluating method determines a fail result in the number of fields of view pass/fail determination process, these processes are executed again with the addition of a micrograph acquired in the input process.

The input process (step S31), the phase classification process (step S32), and the quantitative value calculation process (step S33), are each the same as the process with the same name according to the second embodiment.

(Magnification determination process)

The magnification determination process is a process in which the magnification determination unit 17 determines a representative magnification (step S34) based on the quantitative values calculated by the quantitative value calculation unit 15.

As described above, the quantitative values of crystal grains of the specified phase preferably include area fraction. The magnification determination unit 17 can determine the magnification as follows.

At a given magnification, when the number of micrographs in which there are two or more crystal grains each exceeding 10 % area fraction exceeds 1/3 of the total number of micrographs at the given magnification acquired in the input process, the magnification determination unit 17 adopts a smaller magnification. This is because a micrograph in which two or more crystal grains each exceed 10 % area fraction may result in missing grains at the boundaries (periphery) of the micrograph, or the size distribution of the grains may not be correctly evaluated due to the small number of grains imaged. Further, the number exceeding 1/3 of the total number means this is not a feature of some exceptional micrograph, but a trend in micrographs at the given magnification.

Further, at a given magnification, when the number of micrographs in which the crystal grain having the largest area fraction is less than 3 % of the total micrograph area exceeds 1/3 of the total number of micrographs at the given magnification acquired in the input process, the magnification determination unit 17 adopts a larger magnification. This is because a micrograph in which the crystal grain having the largest area fraction is less than 3 % of the total micrograph area has a large number of small crystal grains, and therefore crystal grain shape cannot be properly evaluated. Further, the number exceeding 1/3 of the total number means this is not a feature of some exceptional micrograph, but a trend in micrographs at the given magnification.

In the magnification determination process, the magnification determination unit 17 sets a magnification that satisfies Condition A and Condition B as a representative magnification. Hereinafter, Condition A means, for micrographs at a given magnification, a condition that no more than 1/3 of the total number of micrographs of the given magnification acquired in the input process contains two or more crystal grains each having an area fraction exceeding 10 %. Further, Condition B means, for micrographs at a given magnification, a condition that no more than 1/3 of the total number of micrographs of the given magnification acquired in the input process are such that the crystal grain having the largest area fraction in a micrograph is less than 3 % of the total area of the micrograph.

Here, the upper (10 %) and lower (3 %) limits of area fraction in Conditions A and B of the magnification determination process are examples, are not limited to these examples, and may be any values that allow appropriate evaluation of the size and shape of crystal grains of a target metal material. The quantitative values used in the conditions of the magnification determination process are area fraction in the present embodiment, but may be, for example, ellipsoid size, Feret diameter, or the like. Further, the quantitative values used in conditions of the magnification determination process may be, for example, circularity or number density, when the shape or number of crystal grains has a significant effect on material properties. According to the present embodiment, an example of using area fraction is described because the area fraction of a phase typically affects material properties in many cases.

(First deviation calculation process)

Here, it is possible that there are two or more levels of magnification that satisfy both Condition A and Condition B. When there is a plurality of candidates for representative magnification (Yes in step S35), the first deviation calculation process is carried out according to the present embodiment (step S36). The first deviation calculation process is a process in which the deviation calculation unit 16 calculates deviation of the quantitative values calculated by the quantitative value calculation unit 15. Here, the deviation calculation process for each field of view as in the first embodiment and the second embodiment is referred to as the second deviation calculation process to distinguish from the first deviation calculation process, which calculates the deviation of the quantitative values for each magnification.

The deviation calculation unit 16 calculates the standard deviation of quantitative values for each candidate representative magnification. The magnification with the smallest standard deviation is determined as the representative magnification. Here, when the order of standard deviation magnitude differs according to the quantitative value selected, the magnification with the smallest standard deviation of the quantitative value that is considered important is preferably determined as the representative magnification. For example, area fraction is considered to have the greatest effect on mechanical properties in DP steel sheets. Accordingly, the magnification with the smallest standard deviation for area fraction is preferably determined as the representative magnification. Further, calculating the deviation of all quantitative values calculated in the quantitative value calculation process is not necessary. For example, deviation need not be calculated for quantitative values that have little impact on the properties of a metal material.

When the first deviation calculation process is executed, the magnification determination process is executed again. In the second magnification determination process, the magnification determination unit 17 determines one final representative magnification from the already selected candidate representative magnifications based on the deviation calculated by the deviation calculation unit 16.

When one representative magnification is determined (No in step S35), the second deviation calculation process is executed (step S37). The second deviation calculation process is the same as that of the deviation calculation process (step S24) according to the second embodiment. The number of fields of view pass/fail determination process (step S38), the statistical value calculation process (step S40), and the output process (step S41) are each the same as the process with the same name according to the second embodiment. Here, the branching (step S39) according to pass/fail of the number of fields of view pass/fail determination process is the same as in step S26 according to the second embodiment.

In this way, the micrograph evaluating method executed by the micrograph evaluating device 10 according to the present embodiment can appropriately and efficiently evaluate the number of fields of view of micrographs of a metal material. A result of evaluation is fed back to an observer to determine an appropriate number of fields of view for observing metallic structure, independently of subjectivity of the observer. Further, an observer does not have to take unnecessarily large numbers of micrographs while changing the field of view, and therefore work efficiency can be improved. Further, the output of a statistical value outside of the micrograph evaluating device 10 allows for verification or secondary pass/fail judgment and the like, regarding the evaluation executed by the micrograph evaluating device 10. Further, the magnification-determination unit 17 can objectively and efficiently determine a representative magnification from a plurality of micrographs of a metal material.

(EXAMPLES)

Advantageous effects of the present disclosure are described in detail below based on examples, although the present disclosure is not limited to these examples. In the examples, the micrograph evaluating method was performed by the micrograph evaluating device 10 according to the first embodiment.

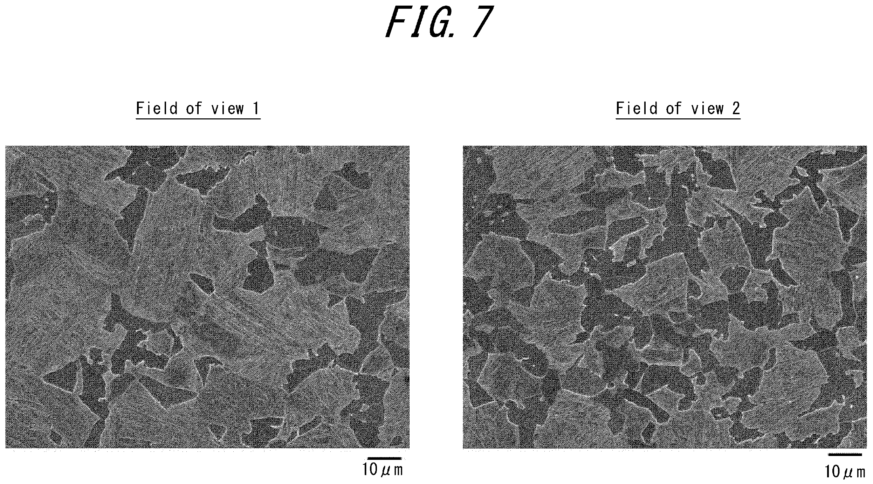

DP steel sheets consisting of ferrite phase and martensite phase were rough polished, and after finish polishing with diamond paste, the steel sheets were etched with nital. Micrographs were then taken for two random fields of view using an electron microscope at a magnification of 1000$\times$. The plurality of micrographs taken were acquired (input process). FIG. 7 illustrates a portion of the acquired micrographs. Here, the phase indicated by white contrast is martensite and the phase indicated by black contrast is ferrite.

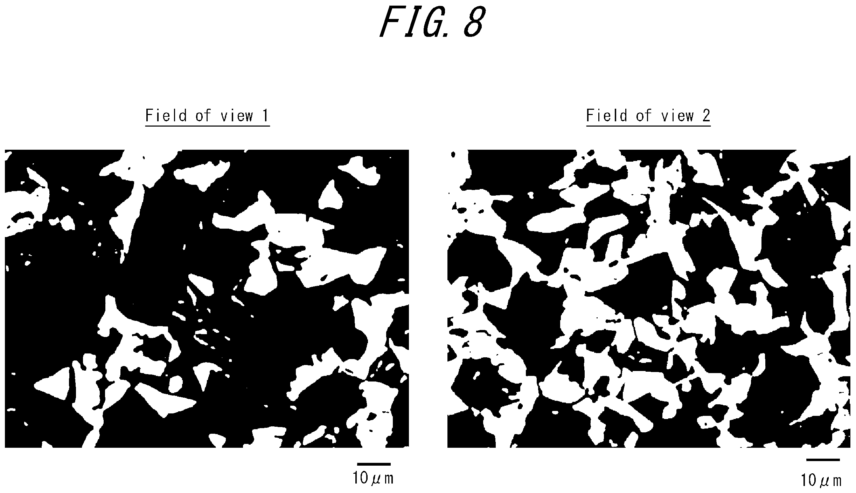

Next, the acquired micrographs were classified into ferrite phase and martensite phase as binary images using a random forest model, one of the advanced image interpretation methods described above (phase classification process). FIG. 8 illustrates the micrographs of FIG. 7 after the phase classification process. Here, black indicates martensite phase and white indicates ferrite phase.

Next, area fraction, Feret diameter, and circularity of each crystal grain were calculated as quantitative values for the martensite phase in each micrograph, based on the acquired binarized images (quantitative value calculation process).

Next, normalization was performed on the quantitative values of each micrograph, and deviations and average values of deviations were calculated (deviation calculation process). Table 1 indicates the values of the calculations for each field of view. Here, 0.1 was used as the threshold value in the number of fields of view pass/fail determination. The average deviation of circularity was 0.1 or less. However, the average deviations of area fraction and Feret diameter each exceeded 0.1, and therefore it was determined that the microstructure of the sample could not be appropriately evaluated with two fields of view. That is, a fail determination was indicated in the number of fields of view pass/fail determination (number of fields of view pass/fail determination process). Here, in Table 1, and Table 2 and Table 3 below, Feret diameter indicates average Feret diameter per micrograph.

[Table 1]

(Table 1)

| Field of view (ID No.) | Area fraction [%] | Normalized area fraction [-] | Deviation of area fraction [-] | Circularity [-] | Normalized circularity [-] | Deviation of circularity [-] | Feret diameter [$\mu$m] | Normalized Feret diameter [-] | Deviation of Feret diameter [-] |
|---|---|---|---|---|---|---|---|---|---|
| 1 | 76 | 1.10 | 0.10 | 0.75 | 1.07 | 0.07 | 126 | 1.36 | 0.36 |
| 2 | 60 | 0.87 | 0.13 | 0.69 | 0.97 | 0.03 | 84 | 0.90 | 0.10 |
| Average deviation | - | - | 0.12 | - | - | 0.05 | - | - | 0.23 |

EP 4 481 379 A1

Therefore, the classification process, the quantitative value calculation process, and the deviation calculation process were repeated by taking additional micrographs with different fields of view until the number of fields of view passed the number of fields of view pass/fail determination process. Table 2 indicates the results for a total of three fields of view. Further, Table 3 indicates the results for a total of nine fields of view.

[Table 2]

(Table 2)

| Field of view (ID No.) | Area fraction [%] | Normalized area fraction [-] | Deviation of area fraction [-] | Circularity [-] | Normalized circularity [-] | Deviation of circularity [-] | Feret diameter [$\mu$m] | Normalized Feret diameter [-] | Deviation of Feret diameter [-] |
|---|---|---|---|---|---|---|---|---|---|
| 1 | 76 | 1.10 | 0.10 | 0.75 | 1.07 | 0.07 | 126 | 1.36 | 0.36 |
| 2 | 60 | 0.87 | 0.13 | 0.69 | 0.97 | 0.03 | 84 | 0.90 | 0.10 |
| 3 | 68 | 0.99 | 0.01 | 0.75 | 1.07 | 0.07 | 92 | 0.98 | 0.02 |
| Average deviation | - | - | 0.08 | - | - | 0.05 | - | - | 0.16 |

[Table 3]

[Table 3]

(Table 3)

| Field of view (ID No.) | Area fraction [%] | Normalized area fraction [-] | Deviation of area fraction [-] | Circularity [-] | Normalized circularity [-] | Deviation of circularity [-] | Feret diameter [µm] | Normalized Feret diameter [-] | Deviation of Feret diameter [-] |
|---|---|---|---|---|---|---|---|---|---|
| 1 | 76 | 1.10 | 0.10 | 0.75 | 1.07 | 0.07 | 126 | 1.36 | 0.36 |
| 2 | 60 | 0.87 | 0.13 | 0.69 | 0.97 | 0.03 | 84 | 0.90 | 0.10 |
| 3 | 68 | 0.99 | 0.01 | 0.75 | 1.07 | 0.07 | 92 | 0.98 | 0.02 |
| 4 | 73 | 1.06 | 0.06 | 0.72 | 1.02 | 0.02 | 97 | 1.04 | 0.04 |
| 5 | 70 | 1.01 | 0.01 | 0.74 | 1.05 | 0.05 | 82 | 0.89 | 0.11 |
| 6 | 68 | 0.98 | 0.02 | 0.66 | 0.93 | 0.07 | 87 | 0.94 | 0.06 |
| 7 | 65 | 0.95 | 0.05 | 0.69 | 0.97 | 0.03 | 88 | 0.94 | 0.06 |
| 8 | 69 | 1.00 | 0.00 | 0.69 | 0.98 | 0.02 | 97 | 1.04 | 0.04 |
| 9 | 71 | 1.04 | 0.04 | 0.65 | 0.92 | 0.08 | 85 | 0.92 | 0.08 |
| Average deviation | - | - | 0.05 | - | - | 0.05 | - | - | 0.10 |

When the total number of fields of view was three, the average deviations of area fraction and circularity were 0.1 or less. However, the average deviation of Feret diameter exceeded 0.1, and therefore it was determined that the microstructure of the sample could not be appropriately evaluated with three views. That is, a fail determination was indicated in the number of fields of view pass/fail determination.

When the total number of fields of view was nine, the average deviations for all quantitative values was 0.1 or less, and therefore a pass determination was indicated in the number of fields of view pass/fail determination. Therefore, the micrograph evaluating device 10 indicated that the microstructure of the sample could be appropriately evaluated by imaging nine fields of view.

Here, as a conventional method of a Comparative Example, the number of fields of view was determined subjectively by an observer for the same sample. According to the Comparative Example, the observer determined that the microstructure could be evaluated in ten fields of view. However, according to the Comparative Example, more than ten fields of view were imaged, and taking and determining the number of fields of view took a long time. The present device is capable of efficient imaging, and determination is completed nearly instantly.

Thus, according to the present Example, the number of fields of view of micrographs of a metal material was appropriately and efficiently evaluated. As a result, correlation with material properties can be objectively considered, and efficient material development can be expected. Further, quantitative values of each phase can be evaluated appropriately in the evaluation of steel material or metal material with multiple metallic phases, and therefore development of efficient steel material or metal material with multiple metallic phases may be expected.

As described above, the micrograph evaluating method, the micrograph evaluating device, the imaging device, and the program according to the present embodiment can appropriately and efficiently evaluate the number of fields of view of micrographs of a metal material.

Although embodiments of the present disclosure have been described based on the drawings and examples, it should be noted that a person skilled in the art may make variations and modifications based on the present disclosure. Therefore, it should be noted that such variations and modifications are included within the scope of the present disclosure. For example, functions and the like included in each component and step may be rearranged, and a plurality of components and steps may be combined into one or divided, as long as no logical inconsistency results. The embodiments according to the present disclosure may be realized as a storage medium on which a program executed by a processor provided to a device is stored. The scope of the present disclosure should be understood to include these examples.

REFERENCE SIGNS LIST

| | |
|---|---|
| 1 | micrograph evaluating system |
| 10 | micrograph evaluating device |
| 11 | input interface |
| 12 | output interface |
| 13 | arithmetic section |
| 14 | phase classification unit |
| 15 | quantitative value calculation unit |
| 16 | deviation calculation unit |
| 17 | magnification determination unit |
| 19 | number of fields of view pass/fail determination unit |
| 20 | statistical value calculation unit |
| 30 | imaging device |

**Claims**

**1.** A micrograph evaluating method comprising:

> an input process of acquiring a plurality of micrographs taken at a defined magnification for two or more fields of view of a metal material;
> a phase classification process of classifying phases in the plurality of micrographs;
> a quantitative value calculation process of calculating quantitative values of the phases classified;
> a deviation calculation process of calculating deviation of the quantitative values;
> a number of fields of view pass/fail determination process of determining a pass/fail result for a number of fields of view of the plurality of micrographs, based on the deviation; and
> an output process of outputting the pass/fail result.

2.  A micrograph evaluating method comprising:

    an input process of acquiring a plurality of micrographs taken at a defined magnification for two or more fields of view of a metal material;
    a phase classification process of classifying phases in the plurality of micrographs;
    a quantitative value calculation process of calculating quantitative values of the phases classified;
    a deviation calculation process of calculating deviation of the quantitative values;
    a number of fields of view pass/fail determination process of determining a pass/fail result for a number of fields of view of the plurality of micrographs, based on the deviation; and
    a statistical value calculation process of calculating a statistical value based on the quantitative values of the phases; and
    an output process of outputting the statistical value.

3.  A micrograph evaluating method comprising:

    an input process of acquiring a plurality of micrographs taken at two or more magnifications for two or more fields of view of a metal material;
    a phase classification process of classifying phases in the plurality of micrographs;
    a quantitative value calculation process of calculating quantitative values of the phases classified;
    a magnification determination process of determining a representative magnification based on the quantitative values; and
    a deviation calculation process of calculating deviation of the quantitative values for a set of the plurality of micrographs corresponding to the representative magnification;
    a number of fields of view pass/fail determination process of determining a pass/fail result for a number of fields of view of the set of the plurality of micrographs, based on the deviation; and
    a statistical value calculation process of calculating a statistical value based on the quantitative values of the phases; and
    an output process of outputting the statistical value.

4.  The micrograph evaluating method according to claim 2 or 3, wherein the output process outputs the pass/fail result along with the statistical value.

5.  The micrograph evaluating method according to any one of claims 1 to 3, wherein the quantitative values are at least one of area fraction, ellipsoid size, Feret diameter, average diameter, circularity, or number density.

6.  A micrograph evaluating device comprising:

    an input interface configured to acquire a plurality of micrographs taken at a defined magnification for two or more fields of view of a metal material;
    a phase classification unit configured to classify phases in the plurality of micrographs;
    a quantitative value calculation unit configured to calculate quantitative values of the phases classified;
    a deviation calculation unit configured to calculate deviation of the quantitative values;
    a number of fields of view pass/fail determination unit configured to determine a pass/fail result for a number of fields of view of the plurality of micrographs, based on the deviation; and
    an output interface configured to output the pass/fail result.

7.  A micrograph evaluating device comprising:

    an input interface configured to acquire a plurality of micrographs taken at a defined magnification for two or more fields of view of a metal material;
    a phase classification unit configured to classify phases in the plurality of micrographs;
    a quantitative value calculation unit configured to calculate quantitative values of the phases classified;
    a deviation calculation unit configured to calculate deviation of the quantitative values;
    a number of fields of view pass/fail determination unit configured to determine a pass/fail result for a number of fields of view of the plurality of micrographs, based on the deviation; and
    a statistical value calculation unit configured to calculate a statistical value based on the quantitative values of the phases; and
    an output interface configured to output the statistical value.

8. A micrograph evaluating device comprising:

an input interface configured to acquire a plurality of micrographs taken at two or more magnifications for two or more fields of view of a metal material;
a phase classification unit configured to classify phases in the plurality of micrographs;
a quantitative value calculation unit configured to calculate quantitative values of the phases classified;
a magnification determination unit configured to determine a representative magnification based on the quantitative values; and
a deviation calculation unit configured to calculate deviation of the quantitative values for a set of the plurality of micrographs corresponding to the representative magnification;
a number of fields of view pass/fail determination unit configured to determine a pass/fail result for a number of fields of view of the set of the plurality of micrographs, based on the deviation; and
a statistical value calculation unit configured to calculate a statistical value based on the quantitative values of the phases; and
an output interface configured to output the statistical value.

9. An imaging device configured to take the plurality of micrographs acquired by the micrograph evaluating device according to any one of claims 6 to 8.

10. The imaging device according to claim 9, wherein the imaging device is an optical microscope or a scanning electron microscope.

11. A program configured to

cause a computer to function as
an input interface configured to acquire a plurality of micrographs taken at a defined magnification for two or more fields of view of a metal material;
a phase classification unit configured to classify phases in the plurality of micrographs;
a quantitative value calculation unit configured to calculate quantitative values of the phases classified;
a deviation calculation unit configured to calculate deviation of the quantitative values;
a number of fields of view pass/fail determination unit configured to determine a pass/fail result for a number of fields of view of the plurality of micrographs, based on the deviation; and
an output interface configured to output the pass/fail result.

12. A program configured to

cause a computer to function as
an input interface configured to acquire a plurality of micrographs taken at a defined magnification for two or more fields of view of a metal material;
a phase classification unit configured to classify phases in the plurality of micrographs;
a quantitative value calculation unit configured to calculate quantitative values of the phases classified;
a deviation calculation unit configured to calculate deviation of the quantitative values;
a number of fields of view pass/fail determination unit configured to determine a pass/fail result for a number of fields of view of the plurality of micrographs, based on the deviation; and
a statistical value calculation unit configured to calculate a statistical value based on the quantitative values of the phases; and
an output interface configured to output the statistical value.

13. A program configured to

cause a computer to function as
an input interface configured to acquire a plurality of micrographs taken at two or more magnifications for two or more fields of view of a metal material;
a phase classification unit configured to classify phases in the plurality of micrographs;
a quantitative value calculation unit configured to calculate quantitative values of the phases classified;
a magnification determination unit configured to determine a representative magnification based on the quantitative values; and
a deviation calculation unit configured to calculate deviation of the quantitative values for a set of the plurality of micrographs corresponding to the representative magnification;
a number of fields of view pass/fail determination unit configured to determine a pass/fail result for a number of fields of view of the set of the plurality of micrographs, based on the deviation; and
a statistical value calculation unit configured to calculate a statistical value based on the quantitative values of the phases; and
an output interface configured to output the statistical value.

# *FIG. 1*

<u>1</u>

# FIG. 2

```
        ┌─────────────┐
        │    Start    │
        └─────────────┘
               │
               ▼
   ┌───────────────────────────┐
   │      Input process        │──S11
   └───────────────────────────┘
               │
               ▼
   ┌───────────────────────────┐
   │ Phase classification      │──S12
   │        process            │
   └───────────────────────────┘
               │
               ▼
   ┌───────────────────────────┐
   │ Quantitative value        │──S13
   │  calculation process      │
   └───────────────────────────┘
               │
               ▼
   ┌───────────────────────────┐
   │ Deviation calculation     │──S14
   │        process            │
   └───────────────────────────┘
               │
               ▼
   ┌───────────────────────────┐
   │ Number of fields of view  │──S15
   │ pass/fail determination   │
   │        process            │
   └───────────────────────────┘
               │                          S16
               ▼                     No
        ◇─────────────◇─────────────────────┐
         ╲   Pass?    ╱                      │
          ◇─────────◇                        │
               │ Yes                         │
               ▼                             │
   ┌───────────────────────────┐            │
   │     Output process        │──S17       │
   └───────────────────────────┘            │
               │                             │
               ▼                             │
        ┌─────────────┐                      │
        │     End     │                      │
        └─────────────┘
```

26

# FIG. 3

## FIG. 4

```
                    ┌─────────┐
                    │  Start  │
                    └─────────┘
                         │
         ┌───────────────┼──────────────────┐
         ▼                                   │
  ┌──────────────────────────────┐          │
  │        Input process         │~S21      │
  └──────────────────────────────┘          │
                 │                           │
                 ▼                           │
  ┌──────────────────────────────┐          │
  │  Phase classification process│~S22      │
  └──────────────────────────────┘          │
                 │                           │
                 ▼                           │
  ┌──────────────────────────────┐          │
  │Quantitative value calculation process│~S23
  └──────────────────────────────┘          │
                 │                           │
                 ▼                           │
  ┌──────────────────────────────┐          │
  │  Deviation calculation process│~S24     │
  └──────────────────────────────┘          │
                 │                           │
                 ▼                           │
  ┌──────────────────────────────┐          │
  │  Number of fields of view pass/│~S25    │
  │  fail determination process  │          │
  └──────────────────────────────┘          │
                 │                     S26   │
                 ▼                      No    │
         ◄───── Pass? ─────►───────────────┘
                 │
                 │ Yes
                 ▼
  ┌──────────────────────────────┐
  │Statistical value calculation process│~S27
  └──────────────────────────────┘
                 │
                 ▼
  ┌──────────────────────────────┐
  │        Output process        │~S28
  └──────────────────────────────┘
                 │
                 ▼
            ┌─────────┐
            │   End   │
            └─────────┘
```

# FIG. 5

*FIG. 6*

```
                    ( Start )
                        │
                        ▼
            ┌──────────────────────────┐
            │      Input process       │─── S31
            └──────────────────────────┘
                        │
                        ▼
            ┌──────────────────────────┐
            │ Phase classification process │─── S32
            └──────────────────────────┘
                        │
                        ▼
            ┌──────────────────────────────┐
            │ Quantitative value calculation process │─── S33
            └──────────────────────────────┘
                        │
                        ▼
            ┌──────────────────────────────┐
            │ Magnification determination process │─── S34
            └──────────────────────────────┘
                        │                      S35
                        ▼
  No  ◇ Plurality of representative magnification candidates? ◇
                        │ Yes
                        ▼
            ┌──────────────────────────────┐
            │ First deviation calculation process │─── S36
            └──────────────────────────────┘
                        │
                        ▼
            ┌──────────────────────────────┐
            │ Second deviation calculation process │─── S37
            └──────────────────────────────┘
                        │
                        ▼
            ┌──────────────────────────────┐
            │ Number of fields of view pass/ │─── S38
            │ fail determination process   │
            └──────────────────────────────┘
                        │                      S39
                        ▼
          ◇          Pass?          ◇ No
                        │ Yes
                        ▼
            ┌──────────────────────────────┐
            │ Statistical value calculation process │─── S40
            └──────────────────────────────┘
                        │
                        ▼
            ┌──────────────────────────┐
            │      Output process      │─── S41
            └──────────────────────────┘
                        │
                        ▼
                    ( End )
```

# FIG. 7

Field of view 1

Field of view 2

10 μm

10 μm

EP 4 481 379 A1

*FIG. 8*

Field of view 1

Field of view 2

10 μm

10 μm

## INTERNATIONAL SEARCH REPORT

International application No.

**PCT/JP2023/013410**

### A. CLASSIFICATION OF SUBJECT MATTER

*G01N 33/20*(2019.01)i; *G01N 1/28*(2006.01)i
FI: G01N33/20; G01N1/28 F

According to International Patent Classification (IPC) or to both national classification and IPC

### B. FIELDS SEARCHED

Minimum documentation searched (classification system followed by classification symbols)

G01N33/20; G01N1/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | WO 2022/059186 A1 (TOSHIBA KK) 24 March 2022 (2022-03-24) fig. 1-17, paragraphs [0015], [0034]-[0049] | 1-13 |
| A | JP 2009-287941 A (NIPPON STEEL CORP) 10 December 2009 (2009-12-10) entire text, all drawings | 1-13 |
| A | JP 2019-012037 A (JFE STEEL CORP) 24 January 2019 (2019-01-24) entire text, all drawings | 1-13 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **31 May 2023** | **13 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/013410**

| Patent document<br>cited in search report | Publication date<br>(day/month/year) | Patent family member(s) | Publication date<br>(day/month/year) |
|---|---|---|---|
| WO 2022/059186 A1 | 24 March 2022 | US 2022/0318983 A1<br>fig. 1-17 | |
| JP 2009-287941 A | 10 December 2009 | (Family: none) | |
| JP 2019-012037 A | 24 January 2019 | (Family: none) | |

Form PCT/ISA/210 (patent family annex) (January 2015)

**EP 4 481 379 A1**

**Patent documents cited in the description**

- JP 2009287941 A